Europäisches Patentamt

European Patent Office

Office européen des brevets

(11) Publication number: **0 334 516**
**A2**

## EUROPEAN PATENT APPLICATION

(21) Application number: **89302385.3**

(22) Date of filing: **10.03.89**

(51) Int. Cl.⁴: **A61D 7/00 , A61K 9/22**

(30) Priority: **21.03.88 US 170495**

(43) Date of publication of application:
**27.09.89 Bulletin 89/39**

(84) Designated Contracting States:
**AT BE CH DE ES FR GB GR IT LI LU NL SE**

(71) Applicant: PFIZER INC.
235 East 42nd Street
New York, N.Y. 10017(US)

(72) Inventor: **Fournier, Loreen Ann**
**80-7, Buddington Road**
**Groton Connecticut(US)**
Inventor: **Lo, Jeelin**
**20, Stagecoach Road**
**Old Lyme Connecticut(US)**

(74) Representative: **Bradbrook, Geoffrey William**
**PFIZER LIMITED Ramsgate Road**
**Sandwich Kent(GB)**

(54) Method for enhancing the unrolling of a rolled matrix device.

(57) A method for enhancing the unrolling of an active agent-containing matrix device, including a laminate device, in an aqueous use environment, said device being constrained in a rolled configuration prior to placement in said environment, comprising subjecting the device to an annealing step prior to constraining it in a rolled configuration; and the devices so treated.

EP 0 334 516 A2

# METHOD FOR ENHANCING THE UNROLLING OF A ROLLED MATRIX DEVICE

This invention relates to a method for enhancing the unrolling of a controlled release active agent-containing polymer matrix device in an aqueous environment, the device being constrained in a rolled configuration prior to placement in said environment. More particularly, it relates to a method for enhancing the unrolling of such an active agent-containing polymer matrix device, including a laminate device, in an aqueous environment, the device being constrained in a rolled configuration prior to placement in said environment which comprises subjecting the device to an annealing step prior to constraining it in a rolled configuration.

Administration of an active agent to a use environment in such a manner that the agent is released in a controlled manner over a prolonged period of time is well known in the veterinary pharmaceutical and medical arts. A large variety of devices has been developed for achieving the desired result. Representative of such devices of value for use in a mammalian system, and especially in ruminant animals, are variable geometry devices of the polymer matrix type. Particularly valuable for use in ruminant animals are matrix type devices having a size and composition which permit them to be constrained to a size and shape suitable for oral administration to a mammal and which, when in the use environment, revert to their original or near-original pre-constrained shape. Included in the term matrix devices as used herein are laminated matrix devices.

Variable geometry matrix devices of the type referred to above are disclosed in U.S. Patent 4,228,149, issued October 14, 1980. Laminate devices comprising an active agent containing resilient polymeric sheet and a polymer film coating one or both sides of said sheet are also disclosed in said patent. Perforated laminate devices are described in U.S. Patent 4,601,893, issued July 22, 1986. The disclosures of each of said patents is incorporated herein by reference.

Preferred matrix devices of the non-laminate type of U.S. 4,228,149, referred to herein as "simple" matrix devices, are those wherein the device comprises a resilient sheet of ethylene vinylacetate (EVA) copolymer as the medium in which the active agent is dispersed. Preferred laminate type devices described in said patent are those wherein the active agent-containing sheet is coated on one or both surfaces with EVA, but not on the edges.

Preferred laminate devices of U.S. 4,601,893 are those wherein the active agent-containing core sheet is EVA. Particularly preferred are those devices wherein the core sheet is coated on both surfaces by films of EVA.

The devices described in the above-cited patents, all oral delivery devices for controlled release of an active agent to a ruminant animal are, as initially manufactured, generally rectangular in shape. To permit their oral administration they are rolled to a cylindrical or substantially cylindrical shape; i.e., a rolled configuration, and constrained in such shape by constraining means. For this reason, the flexibility, and especially the resiliency, of the polymer sheet which comprises the matrix or the core sheet of a laminate device are important as regards the ability of the constrained device to enroll in the use environment, e.g., the rumeno-reticular sac, once the constraining means operates to release the constrained laminate.

Suitable constraining means are, for example, biodegradable string, tape or glue, water soluble adhesive, paper or gelatin capsules.

The herein-described devices can be used to administer a variety of drugs to ruminants. Representative of the active agents which can be used in the devices described herein are anthelmintics, antibacterials, beta-lactams, aminoglycosides, antibacterial growth promotants, antiparasitic agents, essential minerals, vitamins, sulfa drugs, larvacides and insecticides.

The devices described herein offer a practical and especially valuable means for the control (therapeutic and prophylactic) of helminth infections in ruminant animals, cattle in particular. For this purpose anthelmintics, including salts thereof, such as morantel, pyrantel, levamisole, tetramisole, oxantel, ivermectin, piperazine and diethyl carbamazine are of great value.

The constrained devices when placed within the use environment should unroll to their original or near-original rectangular shape so as to permit retention of the device in the rumeno-reticular sac for a prolonged period. However, upon prolonged storage in a constrained shape they tend to set; i.e., to lose their resiliency, and the ability to unroll to a cross-section diameter within a relatively brief period, about two hours, following their administration sufficient to ensure their retention in the rumeno-reticular sac and to prevent regurgitation of the device. Thus, the matrix devices of the art undergo an aging process, which process is a function of time and temperature, which reduces their ability to unroll in the use environment, e.g., the rumen, to a size adequate for retention.

It has now been found that the ability of the herein-described constrained matrix, including laminate, devices to unroll in a use environment even after prolonged periods of storage is markedly enhanced if the devices are subjected to an annealing process prior to their being constrained in a rolled configuration. The annealing process improves the elasticity of the devices such that they recover or unroll from their constrained configuration to a greater extent and at a faster rate. The term "annealing" as used herein is intended to mean "to temper by heating".

Included within the scope of this application are matrix, including laminate, devices of the type described above which have been subjected to tensile stress prior to being constrained in a rolled configuration. Such stressed devices are reported to require a lesser charge of active agent to achieve a given release rate than do non-stressed devices of like construction. They are disclosed in pending U.S. patent application Serial No. 925,776, filed October 30, 1986.

The process of this invention comprises subjecting the devices to an annealing; i.e., a heating step prior to constraining them in a rolled configuration.

The herein-described process is especially useful as regards the devices of U.S. 4,601,893 which comprise morantel or a water soluble acid addition salt of morantel such as the citrate or tartrate. In said devices, the amount of morantel in the core sheet, calculated as acid addition salt can range from 10% to 75% by weight of core sheet.

The annealing temperature can range from about 40°C to about 80°C. A range of about 43° to about 60°C is preferred. The time of annealing varies with the temperature of annealing. Further, the time and temperature vary with the manner in which the devices being annealed are arranged for the annealing step. The devices can be punched or cut to their approximate or actual size prior to annealing or the sheets from which the individual devices are punched can be annealed prior to punching out individual devices. The individual devices or sheets can be annealed individually or they can be stacked one upon the other to a height determined only by the size of the annealing equipment. The sheets can also be rolled for ease of handling prior to annealing and punching. In any case, stacking of the devices or sheets or rolling of the sheets generally requires temperatures toward the lower end of the indicated range and times toward the upper end of the range. Each arrangement requires determination of the conditions for optimum annealing. Temperatures which are too high may cause sticking of stacked devices, sheets or rolled sheets. Lower temperatures and longer times are best suited for stacked or rolled materials. For example, a roll of sheet material comprising a core 0.075 inches (1.91 mm) thick and outer layers each 0.05 inch (1.27 mm) thick, rolled on a 14 inch (35.56 cm) spindle to an overall diameter of 94 inches (238.76 cm), is desirably annealed for up to 96 hours at 43°C. Thus, considering the parameters of temperature and overall thickness of the material to be annealed from about 96 to 4 hours is practical.

The simple matrix devices are prepared as described in U.S. 4,228,149. The procedure comprises blending the active agent, e.g. morantel citrate or tartrate, with softened preformed polymer. For example, a strip of polymer is run through a roll mill which is heated to a temperature sufficient to soften the polymer without decomposing the active agent. The active agent is gradually added to the nip of the mill and the softened strip of polymer recirculated until the desired composition and homogeneity are realized. The strip is then formed into sheets of the desired dimensions. The individual sheets are then rolled and constrained in a rolled configuration for storage until use.

The preferred polymer for the simple matrix devices is EVA as described above. When the device is a laminate, EVA is preferred as the core polymer. The outer layer or layers of the laminate can be any polymer which is non-toxic to mammals and which is stable to the conditions of annealing. As noted previously, the laminate devices comprising EVA as the only polymer are particularly preferred.

The dimensions of the sheets prior to their being constrained depend, of course, upon the ruminant animals for which they are intended. For bovines the overall dimensions of thickness, length and width range from 2-4 mm, 5-15 cm and 4-10 cm, respectively. For sheep suitable dimensions are from 1-3 mm, 5-10 cm and 3-8 cm, respectively. The sheets are rolled and constrained in the form of cylinders for storage and administration to ruminants. In general, the height of the cylinder equals the width of the unrolled or open sheet. The diameters of constrained devices for use in bovines are from 2.0 to 3.0 cm in diameter. Those for use in sheep are from 0.8 to 1.5 cm in diameter.

The constrained devices for use in ruminants must unroll in the rumeno-reticular sac to a cross-section diameter sufficient to prevent regurgitation. Experience has demonstrated this degree of unrolling should occur within a period of about 2 hours post dosing.

The active agent-containing layer, e.g. the core, of laminate device, is prepared in sheets as described above. The core sheet is then sandwiched between films of polymer which comprise the outer layers of the laminate. They are bonded to the core sheet by applying sufficient pressure and heat to achieve bonding without destroying the active agent or the laminate. The laminated sheet is then cut to the desired dimensions. Before or after cutting perforation of the desired size and arrangement can be made by known techniques. The device is then rolled and constrained.

Further, if the herein-described devices are to be subjected to tensile stress, this step, in the case of a simple matrix device, is conducted on the finished sheet prior to rolling. In the case of a laminate device it can be carried out on the core sheet alone, but is preferably carried out on the laminate sheet, before or after perforations are made. The tensile stress must not exceed the yield point of the polymer matrix. The stress is normally applied in only one direction, usually along the length. It can, however, be applied along the width or the bias (diagonal), or along any of said directions in step-wise or concurrent fashion. The laminate is then cut to the desired dimensions. Before or after cutting of the laminate, perforations of the desired size and arrangement are made therein by known techniques. (Porosigens, if used, are blended into the core sheet along with the desired substance).

It will be noted that while the matrix devices are heated during the process of their preparation, the level and time of heating cannot be looked upon as an annealing step. The prior art devices, e.g. those of U.S. 4,228,149 and U.S. 4,601,893, are subjected to heat during preparation and yet the finished devices are subject to a tendency to set up on long term storage or on simulated long term storage, e.g. heating at 40°C for 48 hours. It is only after being subjected to the annealing process of this invention that they retain their ability to unroll in a use environment even after long term storage. The devices thus treated afford an important advantage over devices not subjected to an annealing step.

The unrolling of an annealed perforated laminate device, whether or not subjected to tensile stress, depends to some extent upon the number of holes in the device. A device having 70 holes tends to unroll to a greater extent in a two hour period than a device having 55 or less holes.


### EXAMPLE 1

Equal weight of morantel tartrate and EVA (Type MU-760 having 19% vinyl acetate content, available from U.S.I. Chemicals Co., 99 Park Ave., New York, NY 10016) were mixed via a blend/mill, blend operation using a drum roller and Fitzpatrick D mill with number 2 plate at medium speed and knives forward to obtain a blend containing 50/50 (w/w) blend of the materials. This blend was compounded into a sheet using a Banbury mixer and then dried into small particles for extrusion. The material was extruded using 88,96,99°C as the zone temperatures and 105°C as its die temperature. The screw speed was 40 RPM. The resultant sheet was approximately 0.060 (0.152 cm) thick and 6 inches (15.24 cm) wide. The extruded material was cut into sheets 9 inches (22.86 cm) long and 4.25 inches (10.795 cm) wide and dip-coated three times in a 10% EVA solution in toluene and dried at 50°C. The sheets were divided into two batches. One set was punched to symmetrically place 55 holes (2.7 mm diameter) through the sheets and the other to symmetrically place 70 (2.7 mm) holes through the sheets.

Sheets thus prepared were placed in an annealing oven. One group of seven sheets from each batch was annealed at 50°C for 24 hours. The annealed sheets, along with 7 control (non-annealed) sheets were constrained in a rolled configuration using a water soluble adhesive tape. All were then heated at 40°C for 48 hours to simulate long term storage at room temperature.

The devices were then placed in 40°C water and the degree of unrolling of the individual devices determined by measuring the cross-section diameter of the devices in each group at varying time intervals.

| Comparison of Unrolling (in cm) of Devices with Different Numbers of Holes | | | | |
|---|---|---|---|---|
| Time (Min) | 55 Hole Control | 55 Hole 24 Hrs 50°C | 70 Hole Control | 70 Hole 24 Hrs 50°C |
| 0 | 2.6 | 2.6 | 2.5 | 2.7 |
| 10 | 3.4 | 4.5 | 3.4 | 5.5 |
| 30 | 3.8 | 5.5 | 4.0 | 6.2 |
| 45 | 3.9 | 5.8 | 4.2 | 6.7 |
| 60 | 4.1 | 6.1 | 4.3 | 7.1 |
| 90 | 4.2 | 6.7 | 4.5 | 7.7 |
| 120 | 4.2 | 7.0 | 4.7 | 8.1 |

The annealed devices unrolled at a faster rate and to a greater extent than the unannealed control.

4

Under a given set of annealing conditions a laminate device as described herein having 70 holes symmetrically placed through it unrolled to a somewhat greater extent in a two hour period than a similar device having 55 holes.

## EXAMPLE 2

Following the procedure of Example 1, devices were made in which no holes were punched. The extent of unrolling at various time intervals is given below:

| Sample\Time | Maximum Diameter Unrolled (Cm) | | | | | | | |
|---|---|---|---|---|---|---|---|---|
| | 0 | 10 | 30 | 45 | 60 | 90 | 120 | min. |
| Control 1 | 2.6 | 3.2 | 4.0 | 4.2 | 4.3 | 4.4 | 4.5 | |
| Control 2 | 2.6 | 3.4 | 3.9 | 4.0 | 4.1 | - | 4.3 | |
| A, 50°C/ 24 hours | 2.6 | 5.2 | 6.2 | 6.5 | 6.9 | 7.5 | 7.7 | |
| B, 60°C/ 8 hours | 2.6 | 5.2 | 6.0 | 6.4 | 6.9 | - | 7.4 | |

Controls:   no annealing

Each of the annealed samples unrolled to a greater extent and at a faster rate than the control samples. The differences in annealing conditions of the two samples A and B gave comparable unrolling effects.

## EXAMPLE 3

Twelve 70 hole laminate devices of the type described in Example 1 were divided into two lots of six devices each. Lot A was annealed at 50°C for 24 hours and lot B at 50°C for 48 hours. Their rates and extent of unrolling are presented below.

| Sample\Time | 0 | 10 | 30 | 45 | 60 | 90 | 120 | min. |
|---|---|---|---|---|---|---|---|---|
| Lot A 50°C/24 hours | 2.7 | 5.5 | 6.2 | 6.7 | 7.1 | 7.7 | 8.1 | |
| Lot B 50°C/48 hours | 2.6 | 5.5 | 6.5 | 6.9 | 7.5 | 8.0 | 8.4 | |

For a given annealing temperature (50°C), no significant benefit in unrolling was realized by the longer annealing period.

## EXAMPLE 4

Devices of this invention comprising a laminated device having morantel tartrate as the active agent and laminae of EVA was prepared as follows.

A sheet containing 50% morantel tartrate and 50% EVA (Type MU-760 having 19% vinyl acetate

5

content, available from U.S.I. Chemicals Co., 99 Park Ave., New York, NY 10016) was prepared by dry blending 15 kg morantel tartrate and 15 kg EVA in a 55 gallon (208 liter) fiber drum on a drum roller for 30 minutes. It was then milled at high speed to produce a mixture which passed through a 0.033 inch (0.0838 cm) screen and then drum rolled for another 30 minutes.

The resulting mixture was extruded on a $1\frac{1}{4}$ (3.75 cm) single screw extruder with three heating zones and a 24:1 1:d general purpose screw. The three heating zones of the extruder barrel were set to 88°, 102° and 107° C and the screw was operated at 79 rpm. The die was a 6 inch (15.24 cm) adjustable sheet die opened to 0.110 inch (0.279 cm) and with temperature set to 88° C. The extruded sheet was passed through a three roll take-up with chilled rolls and collected.

The extruded sheet was then coated with EVA UE-633 (available from U.S.I.) by extrusion coating; a thickness of 0.005" (0.127 mm) being applied first to one side and then to the other side by standard methodology.

5.08 x 25.4 cm pieces of coated sheet were then subjected to various tensile strain levels at a strain rate of 5 cm/minute using a standard Instron machine. A total of 33 holes (2.7 mm, diameter) were punched into each device. It was noted that 95% to 100% of the imposed strain was recovered.

The sheets were then annealed at 50° C for 24 hours then constrained in a rolled configuration and subjected to 40° C for 48 hours to simulate long term storage at room temperature. Devices thus made are found to unroll at a faster rate and to a greater extent than do unannealed controls.

## EXAMPLE 5

A mixture of EVA and morantel tartrate (50/50 by weight) was extruded once in order to obtain a uniform blend. The extrudant was chopped and then re-extruded to obtain a 734 mm (28.9 inches) wide and 1.9 mm (0.075 inch) thick sheet. An EVA film of 0.127 mm (0.005 inch) thickness was laminated first on one side and then on the other side of the sheet by an extrusion/compression process. The laminated sheet was trimmed to 622.3 mm (24.5 inches) wide and then slit into 3 strips of 206 mm (8.1 inches) wide each. Each strip was passed through a puncher and then through a cutter to obtain individual devices having 40, 55 or 70 holes of 2.7 mm (0.106 inch) diameter. The dimensions of each device were 20.8 cm (8.187 inches) long, 9.5 cm (3.75 inches) wide and 2.16 mm (0.085 inch) thick.

Devices were placed vertically on their long sides and were annealed at a temperature of 50° C for 24 hours. They were then rolled and constrained using a water soluble adhesive tape.

## Claims

1. A method for enhancing the unrolling of an active-agent containing polymer matrix device in an aqueous use environment, said device being constrained in a rolled configuration prior to placement in said environment, comprising subjecting the device to an annealing step prior to constraining it in a rolled configuration.

2. A method according to claim 1 wherein the polymer matrix comprises ethylene vinylacetate copolymer and the active-agent comprises morantel or a water-soluble acid addition salt thereof.

3. A method for enhancing the unrolling of an active agent-containing laminate device in an aqueous use environment, said device comprising an active agent-containing polymer matrix sandwiched between polymeric films, said device being constrained in a rolled configuration prior to placement in said environment, comprising subjecting the device to an annealing step prior to constraining it in a rolled configuration.

4. A method according to claim 3 wherein the polymer matrix comprises a mixture of ethylene vinylacetate and morantel or a water-soluble acid addition salt thereof sandwiched between films of ethylene vinylacetate copolymer.

5. A method according to claim 4 wherein the device comprises one or a plurality of macroperforations extending through said films and said polymer matrix.

6. The method according to claim 5 wherein the perimeter edge of the core sheet is sealed by ethylene vinylacetate copolymer.

7. A matrix device for controlled release of an active agent to an aqueous use environment, said device comprising an active agent-containing polymer matrix which has been subjected to an annealing step.

8. A matrix device according to claim 7 wherein the polymer is ethylene vinyl acetate and the active agent is morantel or a water soluble acid addition salt thereof.

9. A matrix device according to claim 8 wherein the device comprises an active-agent-containing polymer matrix sandwiched between polymeric films which are substantially impermeable to the use environment.

10. A matrix device according to claim 9 wherein the polymeric films comprise ethylene vinyl acetate and at least one macroperforation extends through the polymeric films and the matrix.